(19)

**Europäisches Patentamt**
**European Patent Office**
**Office européen des brevets**

(11) **EP 3 785 628 A1**

(12) **EUROPEAN PATENT APPLICATION**

(43) Date of publication:
**03.03.2021 Bulletin 2021/09**

(51) Int Cl.:
***A61B 5/11*** *(2006.01)*

(21) Application number: **19461576.1**

(22) Date of filing: **29.08.2019**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
- **Politechnika Lodzka**
  **90-924 Lodz (PL)**
- **Uniwersytet Medyczny w Lodzi**
  **90-419 Lodz (PL)**
- **Instytut Medycyny Pracy im. prof. dr med. Jerzego**
  **Nofera w Lodzi**
  **91-348 Lodz (PL)**
- **Labo Clinic Sp. z o.o.**
  **10-437 Olsztyn (PL)**
- **Platinum Seed Incubator Sp. z o.o.**
  **87-100 Torun (PL)**

(72) Inventors:
- **AMROZIK, Piotr**
  **97-300 Piotrkow Trybunalski (PL)**
- **MIELCZAREK, Aleksander**
  **94-333 Lodz (PL)**
- **ZAJAC, Piotr**
  **90-215 Lodz (PL)**
- **KOTAS, Rafal**
  **97-400 Belchatow (PL)**
- **MARCINIAK, Pawel**
  **92-524 Lodz (PL)**
- **KAMINSKI, Marek**
  **93-150 Lodz (PL)**
- **SZERMER, Michal**
  **93-352 Lodz (PL)**
- **SAKOWICZ, Bartosz**
  **93-534 Lodz (PL)**

(74) Representative: **Kancelaria Eupatent.pl Sp. z.o.o**
**Ul. Kilinskiego 185**
**90-348 Lodz (PL)**

(54) **A PCB WITH COMPONENTS FOR USE IN DYNAMIC POSTUROGRAPHY DEVICE**

(57) A system for dynamic posturography of a person, the system comprising a wearable device attachable to the person's body and comprising: a printed circuit board (501) on which there are mounted: a MEMS chip (502) comprising a 3-axis inertial sensor (103); an ASIC chip (503) comprising: a data bus (101) communicatively coupled to a memory (104); other components communicatively coupled to the system bus (101) so that they may be managed by a controller (105); a communication module (102) to communicate with an external machine; a clock module (107) configured to provide timing for sampling data from the 3-axis inertial sensor (103) at a preferred rate. The connection paths (504X, 504Y, 504Z) between the MEMS chip (502) and the ASIC chip (503) that transmit measurements of the 3-axis inertial sensor (103) have the same length. The system is configured to execute the following steps: receiving data (201) from a 3-axis inertial sensor (103); filtering of the received data (202); determining angular speed (203) of the wearable device; calculating (204) a projection of a center of gravity onto a base surface, on which a person is positioned wherein $x = h \sin(\alpha)$ and $y = h \sin(\beta)$ wherein h denotes a height at which the device is mounted, while the angles $\alpha$ and $\beta$ are the roll and pitch angles of the angular position of the device calculated based on the data from the 3-axis inertial sensor; calculating (205) movement parameters comprising angular speeds and trajectory and surface areas of the wearable device; and comparing (206) the results of the movement parameters with assumed norms and outputting a result of the comparison as the dynamic posturography parameters.

EP 3 785 628 A1

502

504X

504Y

504Z

505

504G

503

Fig. 5B

**Description**

**TECHNICAL FIELD**

The present invention relates to a printed circuit board (PCB) for use in a dynamic posturography device.

**BACKGROUND OF THE INVENTION**

Posturography is a general term that covers all the techniques used to quantify postural control in upright stance in either static or dynamic conditions. Posturography is also an assessment technique used to objectively quantify and differentiate among a variety of possible sensory, motor, and central adaptive impairments to balance control.

Posturography is therefore a technique used in evaluating and monitoring of persons with balance problems. Results of dynamic postugorraphy measurements can be used as input data to various diagnosis procedures to diagnose balance disorders.

Prior art defines a publication of US20140081177A1 entitled "Posturographic system using a balance board", which discloses a stabilometric system is provided that uses a balance platform to detect problems in the vestibular system via data capture, data visualization and mathematical analysis of data, the system having means for data capture that obtain customized records and store data resulting from the readings of the sensors of the balance platform, means for displaying the data obtained using stabilometric tests on a screen that is controlled by a computer, and means for processing the data obtained from the measurements.

It would be advantageous to provide a dynamic posturography system that would not require an expensive equipment such as the balance platform of the prior art, but a simpler device instead.

The aim of the development of the present invention is an improved PCB for use in a device for dynamic posturography.

**SUMMARY AND OBJECTS OF THE PRESENT INVENTION**

A system for dynamic posturography of a person, the system comprising a wearable device attachable to the person's body and comprising: a printed circuit board on which there are mounted: a MEMS chip comprising a 3-axis inertial sensor; an ASIC chip comprising: a data bus communicatively coupled to a memory; other components communicatively coupled to the system bus so that they may be managed by a controller; a communication module to communicate with an external machine; a clock module configured to provide timing for sampling data from the 3-axis inertial sensor at a preferred rate. The connection paths between the MEMS chip and the ASIC chip that transmit measurements of the 3-axis inertial sensor have the same length, wherein the system is configured to execute the following steps: receiving data from a 3-axis inertial sensor; filtering of the received data; determining angular speed of the wearable device; calculating a projection of a center of gravity onto a base surface, on which a person is positioned wherein $x = h \sin(\alpha)$ and $y = h \sin(\beta)$ wherein h denotes a height at which the device is mounted, while the angles $\alpha$ and $\beta$ are the roll and pitch angles of the angular position of the device calculated based on the data from the 3-axis inertial sensor; calculating movement parameters comprising angular speeds and trajectory and surface areas of the wearable device; comparing the results of the movement parameters with assumed norms and outputting a result of the comparison as the dynamic posturography parameters.

Preferably, the bonding pads of the MEMS chip connected to the connection paths have the same size and the same pitch as the bonding pads of the ASIC chip.

Preferably, the MEMS chip further comprises a magnetometer.

**BRIEF DESCRIPTION OF THE DRAWINGS**

These and other objects of the invention presented herein, are accomplished by providing a system and method for dynamic posturography. Further details and features of the present invention, its nature and various advantages will become more apparent from the following detailed description of the preferred embodiments shown in a drawing, in which:

Fig. 1 presents a diagram of the system according to the present invention;
Fig. 2 presents a diagram of the method according to the present invention;
Fig. 3 presents a simplified diagram of a principle of determining coordinates of a projection of a centre of gravity onto a base plane/surface; and
Fig. 4 presents a method for determining an angular speed;
Figs. 5A-B depict detailed design of a printed circuit board used in a wearable device;
Fig. 6 presents the positions at which the device can be mounted on the person's body.

**NOTATION AND NOMENCLATURE**

Some portions of the detailed description which follows are presented in terms of data processing procedures, steps or other symbolic representations of operations on data bits that can be performed on computer memory. Therefore, a computer executes such logical steps thus requiring physical manipulations of physical quantities.

Usually these quantities take the form of electrical or magnetic signals capable of being stored, transferred, combined, compared, and otherwise manipulated in a computer system. For reasons of common usage, these signals are referred to as bits, packets, messages, values, elements, symbols, characters, terms, numbers, or the like.

Additionally, all of these and similar terms are to be associated with the appropriate physical quantities and are merely convenient labels applied to these quantities. Terms such as "processing" or "creating" or "transferring" or "executing" or "determining" or "detecting" or "obtaining" or "selecting" or "calculating" or "generating" or the like, refer to the action and processes of a computer system that manipulates and transforms data represented as physical (electronic) quantities within the computer's registers and memories into other data similarly represented as physical quantities within the memories or registers or other such information storage.

A computer-readable (storage) medium, such as referred to herein, typically may be non-transitory and/or comprise a non-transitory device. In this context, a non-transitory storage medium may include a device that may be tangible, meaning that the device has a concrete physical form, although the device may change its physical state. Thus, for example, non-transitory refers to a device remaining tangible despite a change in state.

As utilized herein, the term "example" means serving as a non-limiting example, instance, or illustration. As utilized herein, the terms "for example" and "e.g." introduce a list of one or more non-limiting examples, instances, or illustrations.

**DESCRIPTION OF EMBODIMENTS**

The present invention is not based on a balance platform and utilizes an inertia sensor (optionally supported by a magnetic field sensor) allowing for measuring its relative positioning as well as its angular position. Said sensor(s) affixed to a person's body facilitates precise determination of positioning of a selected body part and recording of changes of said positioning in time. A set of such sensors will in turn result in an alternative method for executing dynamic posturography.

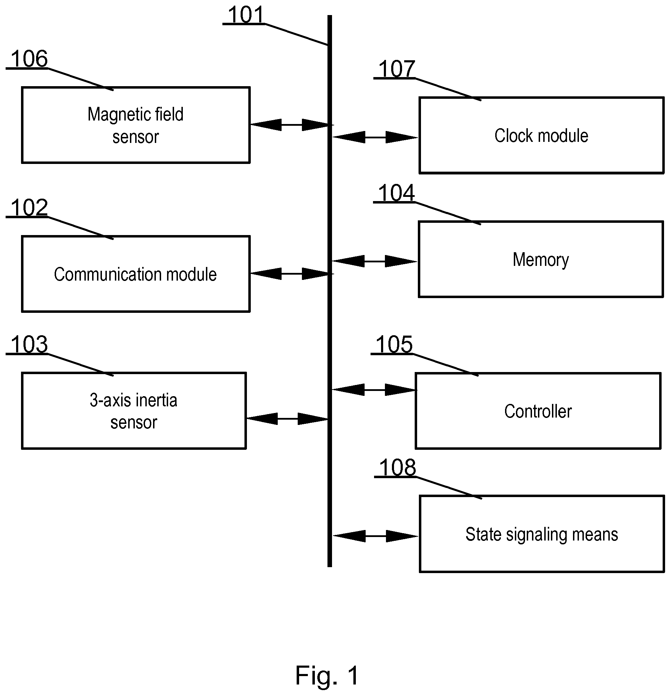

Fig. 1 presents a diagram of the system according to the present invention. The system is preferably supplied with power from a battery and may be realized using dedicated components or custom made FPGA or ASIC circuits. The system comprises a data bus 101 communicatively coupled to a memory 104. Additionally, other components of the system are communicatively coupled to the system bus 101 so that they may be managed by a controller 105.

The memory 104 may store computer program or programs executed by the controller 105 in order to execute steps of the method according to the present invention.

A communication module 102 will typically be user to communicate with an external machine such as a PC, a smartphone, a tablet or the like. The communication link may be such as Wi-Fi, Bluetooth or the like.

The device is preferably sufficiently small and light in order to be mounted on person's body while not hindering any movement of the person.

A 3-axis inertial sensor 103 is implemented in the device and reports data to the controller 105. Such sensor is sometimes referred to as an inertial measurement unit (IMU) and is an electronic device that measures and reports forces, angular rate, and sometimes a level of a surrounding magnetic field (local vector of a magnetic field). The device uses a combination of accelerometers and gyroscopes, sometimes also magnetometers 106.

A clock module 107 may be used for sampling data from the sensors 105, 106 at a preferred rate, which is 20 Hz.

The controller 105 may, at least partially, process the received data from the sensors in order to limit transmission data load.

A signaling means may be present 108 that allow to signal a state of the system to a user. The signaling may be visual or audio wherein visual options may be as simple as LEDs and as sophisticated as small touch screens.

In an alternative embodiment the main sensors pair 103, 106 may be an LSM9DS1 circuit which is supplemented by a reserve LSM6DSM inertia circuit. The reserve inertia circuit is useful because in some cases when in upside - down orientation, the observed levels of noise are greatly increased. Said reserve inertia circuit may be mounted in an opposite orientation to the main sensor thereby allowing for selection of data readings having less noise.

Test embodiments of the present invention have shown that a printed circuit board comprising all the elements of the system may be as small as 3 x 3 cm (excluding the power source).

Typically, the system will operate in a loop comprising general steps of (a) data acquisition, (b) data processing and (c) delivery of results (preferably to a paired external device).

The transmitted data may comprise:

id - identifier of the device, preferably unique identifier;

sno - sample number;

accX - acceleration in the X axis registered by the accelerator of the sensor 103;

accY - acceleration in the Y axis registered by the accelerator of the sensor 103;

accZ - acceleration in the Z axis registered by the accelerator of the sensor 103;

gyroX - angular speed in the X axis of a gyroscope of the sensor 103;

gyroY - angular speed in the Y axis of a gyroscope of the sensor 103;

gyroZ - angular speed in the Z axis of a gyroscope of the sensor 103;

magnX - optionally registered magnetic field in the X axis of a magnetometer sensor 107;

magnY - optionally registered magnetic field in the Y axis of a magnetometer sensor 107;

magnZ - optionally registered magnetic field in the Z axis of a magnetometer sensor 107.

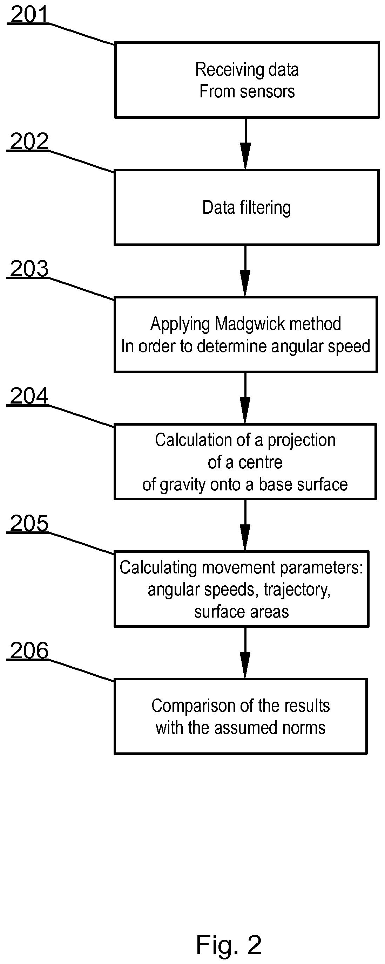

Fig. 2 presents a diagram of the method according to the present invention. The method starts at step 201 from receiving data from sensors 103, 107.

Typically, data from inertia sensors or magnetic field sensors are subject to noise. Therefore, it is necessary to apply data filtering of the input at step 202. According to the present invention, there has been applied a low-pass filter operating in a continuous manner, which preferably is a Butterworth, 2nd-order, digital filter having a cutoff frequency of 10 Hz.

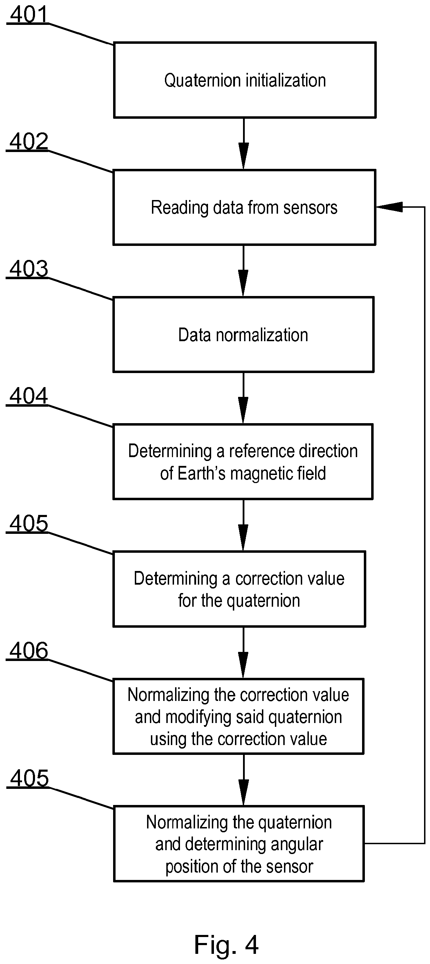

In order to determine angular speed, a particular implementation of a modified Sebastian Madgwick's method has been applied 203, which uses quaternions for representing orientation in three-dimensional space. In mathematics, the quaternions are a number system that extends the complex numbers. They were first described by Irish mathematician William Rowan Hamilton and applied to mechanics in three-dimensional space. The method will be elaborated on in details with reference to Fig. 4.

Sebastian Madgwick's method is based on data from an IMU limited to acceleration and gyroscope data while not taking into account magnetic sensor's data. The method may be divided into the following steps:

- normalization of data from the acceleration sensor;
- gradient descent method application - a corrective step;
- determining a speed of change of the quaternions;
- integration in order to determine quaternions;
- normalization of the quaternions;
- determining an angular position based on the quaternions.

$$\psi = atan2\left(2q_2q_3 - 2q_1q_4, 2q_1^2 + 2q_2^2 - 1\right)$$

$$\theta = -\sin^{-1}\left(2q_2q_4 + 2q_1q_3\right)$$

$$\varphi = atan2\left(2q_3q_4 - 2q_1q_2, 2q_1^2 + 2q_4^2 - 1\right)$$

Based on the calculated quaternions, according to the equations above, there are calculated angles of *roll, pitch, yaw*. They are used in order to determine temporal angular speeds of a rotating rigid body. These are a theoretical creation similar to Euler's angles. They are created by sequential rotations with respect to a base system and not a local system as in case of the Euler's angles. The rotation is effected sequentially against the X axis then Y axis and lastly the Z axis. The order of axes may be different, nevertheless once set it must be followed in subsequent calculations.

At step 204, there is calculated projection of a center of gravity onto a base surface, on which a person is positioned. The equations applied are x=h sin($\alpha$) and y=h sin($\beta$).

At step 205, there are calculated movement parameters such as angular speeds, trajectory, surface areas in the following manner:

- current angular speed in the XY plane

$$\frac{hypot\left(pitch(i) - pitch(i-1), roll(i) - roll(i-1)\right)}{T}$$

where hypot(x,y) is $\sqrt{x^2 + y^2}$

- maximum angular speed in the XY plane
- average angular speed in the XY plane

- trajectory length $\sum_{i=1}^{N} hypot\big(x(i) - x(i-1), y(i) - y(i-1)\big)$ , wherein N is a samples count

- plane area $\sum_{i=1}^{N} \big|\big((x(i-1) - x_0)(y(i) - y_0) - (x(i) - x_0)(y(i-1) - y_0)\big)\big|$ , wherein $x_0$ and $y_0$ is a geometric center of said trajectory.

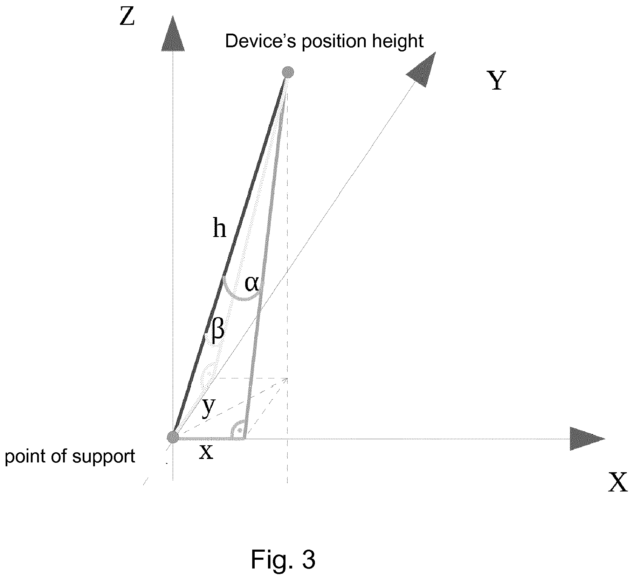

Fig. 3 presents a simplified diagram of a principle of determining coordinates of a projection of a center of gravity onto a base plane/surface.

According to this diagram, the coordinates are calculated based on the following relations:

$$x = h \sin \alpha$$

$$y = h \sin \beta$$

wherein h denotes a height at which the device is mounted, while the angles $\alpha$ and $\beta$ are the previously defined roll and pitch angles.

The results of the measurements are lastly compared with the assumed norms 206. This allows to determine a state of the person under test.

The norms have been determined based on test evaluations, which were also compared with results of a standard static posturography system. The following table presents such norms:

| | Eyes open firm surface | Eyes closed firm surface | Eyes open soft surface | Eyes closed soft surface |
|---|---|---|---|---|
| Max angular speed [deg/s] | 8,82 | 9,07 | 9,93 | 10,41 |
| Avg angular speed [deg/s] | 0,59 | 0,68 | 0,99 | 1,67 |
| COG plane area [cm$^2$] | 2,84 | 2,37 | 14,13 | 68,15 |
| Trajectory length [cm] | 13,57 | 14,40 | 30,21 | 37,31 |

The four tests described above are performed wherein a person runs two tests with eyes open and two test with eyes closes. The first test is performed on a firm surface while the other is performed on a soft surface such as a foam that makes it more difficult to keep a balance.

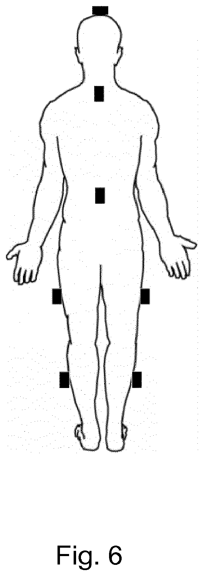

Owing to the use of gyroscopes and magetometers, the device has additional capabilities of registering its positioning. This in turn allows for further analysis (going beyond dynamic posturography) such as: head movements tracking, tracking of legs movements or rotations of the person under test, when the device is attached to a particular position at the person's body, as shown in Fig. 6..

Fig. 4 presents a method for determining an angular speed. At step 401 a quaternion is initialized ($q_0$=1, $q_1$=$q_2$=$q_3$=0).

Subsequently, at step 402, there are read data from the three-axis sensors: accelerometer (a), gyroscope (g) and magnetometer (m). Next 403 the accelerometer data are normalized as well as magnetometer data are normalized. After that, the process moves to step 404, where there is determined a reference direction of Earth's magnetic field by the following calculations:

- $h_x = m_x q_1 q_1 - 2q_1 m_y q_4 + 2q_1 m_z q_3 + m_x q_2 q_2 + 2q_2 m_y q_3 + 2q_2 m_z q_4 - m_x q_3 q_3 - m_x q_4 q_4;$

- $h_y = 2q_1m_xq_4 + m_yq_1q_1 - 2q_1m_zq_2 + 2q_2m_xq_3 - m_yq_2q_2 + m_yq_3q_3 + 2q_3m_zq_4 - m_yq_4q_4$;
- $b_x = hypot(h_x, h_y)/2$
- $b_z = 0.5(-2q_1m_xq_3 + 2q_1m_yq_2 + m_zq_1q_1 + 2q_2m_xq_4 - m_zq_2q_2 + 2q_3m_yq_4 - m_zq_3q_3 + m_zq_4q_4)$;

Subsequently, at step 405 there is determined a correction value for a quaternion based on the current value of the quaternion as well as readings from the sensors. The following equations are used for this purpose:

- $s_1 = - 2q_3 (2q_2q_4 - 2q_1q_3 - a_x) + 2q_2(2q_1q_2 + 2q_3q_4 - a_y) - 2b_zq_3(2b_x (0.5 - q_3q_3 - q_4q_4) + 2b_z(q_2q_4 - q_1q_3) - m_x) + (-2b_xq_4 + 2b_zq_2)(2b_x(q_2q_3 - q_1q_4) + 2b_z(q_1q_2 + q_3q_4) - m_y) + 2b_xq_3(2b_x (q_1q_3 + q_2q_4) + 2b_z(0.5 - q_2q_2 - q_3q_3) - m_z)$
- $s_2 = 2q_4(2q_2q_4 - 2q_1q_3 - a_x) + 2q_1(2q_1q_2 + 2q_3q_4 - a_y) - 4q_2(1 - 2q_2q_2 - 2q_3q_3 - a_z) + 2b_zq_4(2b_x(0.5 - q_3q_3 - q_4q_4) + 2b_z(q_2q_4 - q_1q_3) - m_x) + (2b_xq_3 + 2b_zq_1)(2b_x(q_2q_3 - q_1q_4) + 2b_z(q_1q_2 + q_3q_4) - m_y) + (2b_xq_4 - 4b_zq_2)(2b_x(q_1q_3 + q_2q_4) + 2b_z(0.5 - q_2q_2 - q_3q_3) - m_z)$
- $s_3 = -2q_1(2q_2q_4 - 2q_1q_3 - a_x) + 2q_4(2q_1q_2 + 2q_3q_4 - a_y) - 4q_3(1 - 2q_2q_2 - 2q_3q_3 - a_z) + (-4b_xq_3 - 2b_zq_1)(2b_x(0.5 - q_3q_3 - q_4q_4) + 2b_z(q_2q_4 - q_1q_3) - m_x) + (2b_xq_2 + 2b_zq_4)(2b_x(q_2q_3 - q_1q_4) + 2b_z(q_1q_2 + q_3q_4) - my) + (2b_xq_1 - 4b_zq_3)(2b_x(q_1q_3 + q_2q_4) + 2b_z(0.5 - q_2q_2 - q_3q_3) - m_z)$
- $s_4 = 2q_2(2q_2q_4 - 2q_1q_3 - a_x) + 2q_3(2q_1q_2 + 2q_3q_4 - a_y) + (-4bxq4 + 2b_zq_2) (2b_x(0.5 - q_3q_3 - q_4q_4) + 2b_z(q_2q_4 - q_1q_3) - m_x) + (-2b_xq_1 + 2b_zq_3)(2b_x(q_2q_3 - q_1q_4) + 2b_z(q_1q_2 + q_3q_4) - m_y) + 2b_xq_2(2b_x(q_1q_3 + q_2q_4) + 2b_z(0.5 - q_2q_2 - q_3q_3) - m_z)$

Subsequently, the result of the step 405 is normalized and said quaternion is modified 406 such that:

- $\delta q_1 = 0.5(-q_2g_x - q_3g_y - q_4g_z) - s_1f_s\beta$;
- $\delta q_2 = 0.5(q1g_x + q_3g_z - q_4g_y) - s_2f_s\beta$;
- $\delta q_3 = 0.5(q_1g_y - q_2g_z + q_4g_x) - s_3f_s\beta$;
- $\delta q_4 = 0.5(q_1g_z + q_2g_y - q_3g_x) - s_4f_s\beta$;
- wherein $f_s$- is a sampling frequency, $\beta$ - is an attenuation coefficient (0-1)

Next 407, the quaternion is normalized and used in order to determine angular position of the sensor (in general the angular position is calculated, which depending on needs may be converted to angular speed. In case of calculating distance/length or trajectory plane, angular position may suffice).

Said normalization of the quaternion is preferably based on division of each components by a root of the sum of squares of all components e.g.:

- $q_1=q_1/sqrt(q_1q_1 + q_2q_2 + q_3q_3 + q_4q_4)$

Lastly, the steps 402 - 407 are repeated for next data samples from said sensors in a loop.

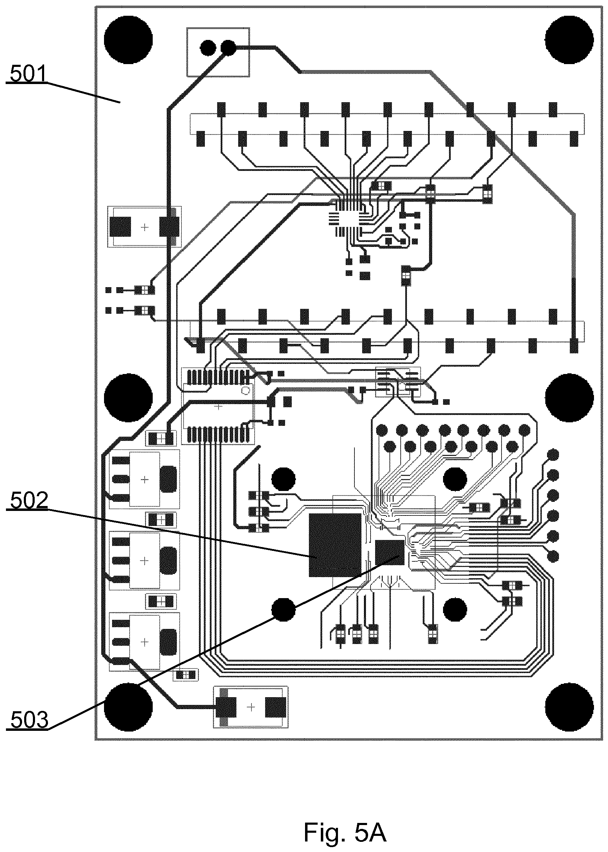

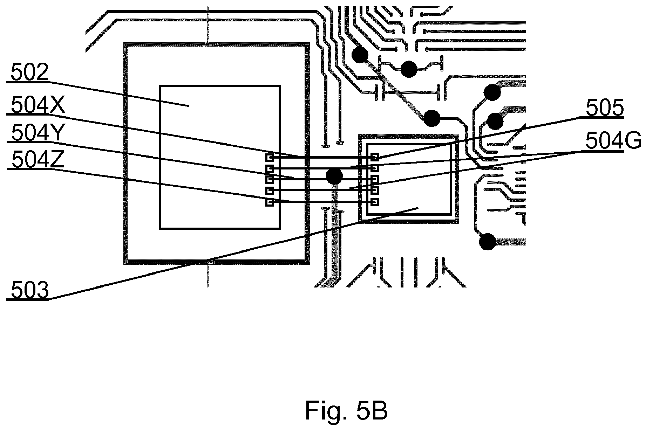

Figs. 5A and 5B depict detailed design of a printed circuit board (PCB) 501 used in a wearable device according to the present invention. Fig. 5A shows the PCB 501, while Fig. 5B shows in enlarged view the area wherein the MEMS and ASIC chips 502, 503 are mounted.

The 3-axis inertial sensor 103 and preferably also the magnetic filed sensor 106 are implemented in a MEMS (Micro-Electro-Mechanical System) chip 502, while the other components 102, 104, 105, 107, 108 of the system are implemented in an ASIC (Application Specific Integrated Circuit) chip 503. Maintaining the symmetry of connections between the MEMS chip 502 with the sensors 103, 106 and the ASIC chip 503 with the other modules 102, 104, 105, 107, 108 is crucial for ensuring the correct readout of the MEMS sensor. The reason is that the MEMS is a differential sensor and its readout signal depends on the difference between two capacitances $(C+\Delta C+C_{p1})$ and $(C-\Delta C+C_{p2})$, where C is the static MEMS capacitance, $\Delta C$ is the capacitance change due to acceleration and $C_{p1}$ and $C_{p2}$ are the parasitic capacitances of the connections between MEMS chip 502 and the ASIC chip 503 (for example due to bonding pad capacitances). Therefore, to keep the readout signal dependent only on $\Delta C$, the design should ensure that $C_{p1}=C_{p2}$, which is only possible if ideal symmetry is kept between MEMS-ASIC connections. Therefore the footprints for MEMS and ASIC chips are positioned in such a way that the connection paths 504X, 504Y, 504Z that transmit measurements of the 3-axis inertial sensor between them have the same length. In addition. in both chips the bonding pads 505 have the same size and the same pitch which allows symmetrical bonding of these two chips 502, 503. Between the connection paths 504X, 504Y, 504Z there may be ground paths 504G.

The aforementioned system and method for dynamic posturography is applied in assessment to quantify the central nervous system adaptive mechanisms involved in the control of posture and balance. Therefore, the invention provides a useful, concrete and tangible result.

The present invention provides a wearable device and a corresponding data processing on input registered by respective sensors. Thus, the machine or transformation test is fulfilled and that the idea is not abstract.

At least parts of the methods according to the invention may be computer implemented. Accordingly, the present invention may take the form of an entirely hardware embodiment, an entirely software embodiment (including firmware, resident software, micro-code, etc.) or an embodiment combining software and hardware aspects that may all generally be referred to herein as a "circuit", "module" or "system".

Furthermore, the present invention may take the form of a computer program product embodied in any tangible medium of expression having computer usable program code embodied in the medium.

It can be easily recognized, by one skilled in the art, that the aforementioned method for dynamic posturography may be performed and/or controlled by one or more computer programs. Such computer programs are typically executed by utilizing the computing resources in a computing device. Applications are stored on a non-transitory medium. An example of a non-transitory medium is a non-volatile memory, for example a flash memory while an example of a volatile memory is RAM. The computer instructions are executed by a processor. These memories are exemplary recording media for storing computer programs comprising computer-executable instructions performing all the steps of the computer-implemented method according the technical concept presented herein.

While the invention presented herein has been depicted, described, and has been defined with reference to particular preferred embodiments, such references and examples of implementation in the foregoing specification do not imply any limitation on the invention. It will, however, be evident that various modifications and changes may be made thereto without departing from the broader scope of the technical concept. The presented preferred embodiments are exemplary only, and are not exhaustive of the scope of the technical concept presented herein.

Accordingly, the scope of protection is not limited to the preferred embodiments described in the specification, but is only limited by the claims that follow.

**Claims**

1. A system for dynamic posturography of a person, the system comprising a wearable device attachable to the person's body and comprising:

    - a printed circuit board (501) on which there are mounted:
    - a MEMS chip (502) comprising a 3-axis inertial sensor (103);
    - an ASIC chip (503) comprising:

        - a data bus (101) communicatively coupled to a memory (104);
        - other components communicatively coupled to the system bus (101) so that they may be managed by a controller (105);
        - a communication module (102) to communicate with an external machine;
        - a clock module (107) configured to provide timing for sampling data from the 3-axis inertial sensor (103) at a preferred rate;

    the system being **characterised in that**:

        - the connection paths (504X, 504Y, 504Z) between the MEMS chip (502) and the ASIC chip (503) that transmit measurements of the 3-axis inertial sensor (103) have the same length;
        - and wherein the system is configured to execute the following steps:

            - receiving data (201) from a 3-axis inertial sensor (103);
            - filtering of the received data (202);
            - determining angular speed (203) of the wearable device;
            - calculating (204) a projection of a center of gravity onto a base surface, on which a person is positioned wherein $x = h \sin(a)$ and $y = h \sin(\beta)$ wherein h denotes a height at which the device is mounted, while the angles $\alpha$ and $\beta$ are the roll and pitch angles of the angular position of the device calculated based on the data from the 3-axis inertial sensor;
            - calculating (205) movement parameters comprising angular speeds and trajectory and surface areas of the wearable device; and
            - comparing (206) the results of the movement parameters with assumed norms and outputting a result of the comparison as the dynamic posturography parameters.

2. The system according to claim 1, wherein the bonding pads (505) of the MEMS chip (502) connected to the connection paths (504X, 504Y, 504Z) have the same size and the same pitch as the bonding pads (505) of the ASIC chip (503).

3. The system according to claim 1 or 2, wherein the MEMS chip (502) further comprises a magnetometer (106).

Fig. 1

201 → Receiving data
From sensors

202 → Data filtering

203 → Applying Madgwick method
In order to determine angular speed

204 → Calculation of a projection
of a centre
of gravity onto a base surface

205 → Calculating movement parameters:
angular speeds, trajectory,
surface areas

206 → Comparison of the results
with the assumed norms

Fig. 2

Fig. 3

401

Quaternion initialization

402

Reading data from sensors

403

Data normalization

404

Determining a reference direction
of Earth's magnetic field

405

Determining a correction value
for the quaternion

406

Normalizing the correction value
and modifying said quaternion
using the correction value

405

Normalizing the quaternion
and determining angular
position of the sensor

Fig. 4

Fig. 5A

502

504X

504Y

504Z

505

504G

503

Fig. 5B

Fig. 6

Europäisches
Patentamt

European
Patent Office

Office européen
des brevets

## EUROPEAN SEARCH REPORT

**Application Number**

EP 19 46 1576

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2017/258386 A1 (WOLTJER REINOUT [NL] ET AL) 14 September 2017 (2017-09-14) * paragraphs [0037], [0068], [0073] * | 1-3 | INV. A61B5/11 |
| Y | Anonymous: "Working with High Speed and RF PCB Trace Lengths in Your Design - ELECT-CAD COMPANY", , 22 July 2019 (2019-07-22), XP055671980, Retrieved from the Internet: URL:http://elect-cadcompany.com/working-with-high-speed-and-rf-pcb-trace-lengths-in-your-design/ [retrieved on 2020-02-27] * the whole document * | 1-3 | |
| Y | US 2019/232113 A1 (ZETS GARY [US] ET AL) 1 August 2019 (2019-08-01) * paragraphs [0015], [0050] - [0071] * | 1-3 | |
| A | US 9 526 946 B1 (ZETS GARY [US] ET AL) 27 December 2016 (2016-12-27) * column 1, line 42 - column 2, line 11 * * column 4, line 36 - column 5, line 3 * * column 9, line 50 - column 11, line 45 * | 1-3 | TECHNICAL FIELDS SEARCHED (IPC) A61B |

The present search report has been drawn up for all claims

| Place of search | Date of completion of the search | Examiner |
|---|---|---|
| Berlin | 27 February 2020 | Trachterna, Morten |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 19 46 1576

This annex lists the patent family members relating to the patent documents cited in the above-mentioned European search report.
The members are as contained in the European Patent Office EDP file on
The European Patent Office is in no way liable for these particulars which are merely given for the purpose of information.

27-02-2020

| Patent document cited in search report | | Publication date | Patent family member(s) | | Publication date |
|---|---|---|---|---|---|
| US 2017258386 | A1 | 14-09-2017 | CN 107106122 A<br>EP 3223758 A1<br>JP 2017535378 A<br>US 2017258386 A1<br>WO 2016085341 A1 | | 29-08-2017<br>04-10-2017<br>30-11-2017<br>14-09-2017<br>02-06-2016 |
| US 2019232113 | A1 | 01-08-2019 | NONE | | |
| US 9526946 | B1 | 27-12-2016 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• US 20140081177 A1